# EUROPEAN PATENT APPLICATION

(11) **EP 4 703 350 A1**
(43) Date of publication of application: **04.03.2026**
(21) Application number: 24796398.6
(22) Date of filing: 24.04.2024
(51) Int. Cl.: C07C 209/36, C07C 211/57, C07C 201/12, C07C 205/06, C07C 205/49, C07D 471/04

(54) **METHOD FOR PREPARING 4-AMINOINDAN AND METHOD FOR PREPARING DIHYDROINDENYL-PYRAZOLO[3,4-B]PYRIDINE-AMINE**

(30) Priority: 25.04.2023 KR 20230054356
(71) Applicant: JW Pharmaceutical Corporation, Gyeonggi-do 13840 (KR)
(72) Inventor: LEE, Sumin, Seoul 06586 (KR); YOON, Dooha, Seongnam-si Gyeonggi-do 13562 (KR); CHIN, Seiho, Hwaseong-si Gyeonggi-do 18297 (KR)
(74) Representative: HGF
(86) International application number: PCT/IB2024/053974
(87) International publication number: WO 2024/224297

(57) **Abstract**

The present invention relates to a method for preparing 4-aminoindan and a method for preparing a dihydroindenyl-pyrazolo[3,4-b]pyridine-amine compound, in which the method for preparing 4-aminoindan comprises the steps of: (Sa) preparing at least one of compound 2a and compound 2b using compound 1; and (Sb) preparing 4-aminoindan using at least one of compound 2a and compound 2b.

## Description

### TECHNICAL FIELD

The present invention relates to a method for preparing 4-aminoindan and a method for preparing a dihydroindenyl-pyrazolo[3,4-b]pyridine amine.

### BACKGROUND ART

4-aminoindan is one of the most widely used compound in the medical and chemical arts per se or as a reactant for the preparation of various compounds. An IUPAC name of 4-aminoindan is 2,3-dihydro-1H-inden-4-amine.

As a method for synthesizing 4-aminoindan, there is disclosed a method for performing a nitration reaction using indan as a starting material to obtain 4-nitroindan, and reducing the same. However, in the nitration reaction according to reaction formula A below (under HNO₃/H₂SO₄ condition), the selectivity for position 5 of indan is higher than that of position 4, and thus 5-nitroindan is rather obtained as a main product, thereby making it difficult to separate and purify 4-nitroindan. In other words, such a method has a problem in that 4-aminoindan is hardly mass-produced at a high yield.

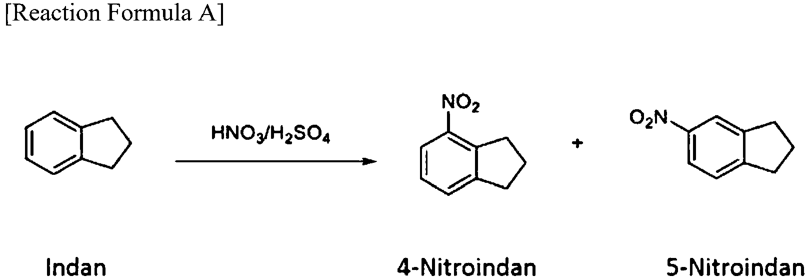

### [Related Art Reference]

### [Patent Documents]

(Patent Document 1) International Publication WO 2019/211463
(Patent Document 2) International Publication WO 2019/212256

### DETAILED DESCRIPTION OF THE INVENTION

### TECHNICAL PROBLEM

One object of the present invention is to provide a method for preparing 4-aminoindan capable of selectively mass-producing only 4-aminoindan at a high yield.

One object of the present invention is to provide a method for preparing a dihydroindenyl-pyrazolo[3,4-b]pyridine amine.

### TECHNICAL SOLUTION

A method for preparing 4-aminoindan for one object of the present invention includes:
(Sa) a step of reacting compound 1 represented by chemical formula 1 to prepare at least one of compound 2a represented by chemical formula 2a and compound 2b represented by chemical formula 2b; and
(Sb) a step of reacting at least one of compounds 2a and 2b to prepare 4-aminoindan represented by chemical formula 3.

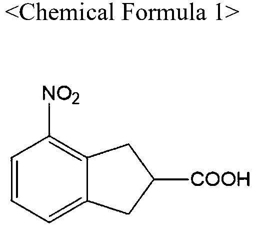

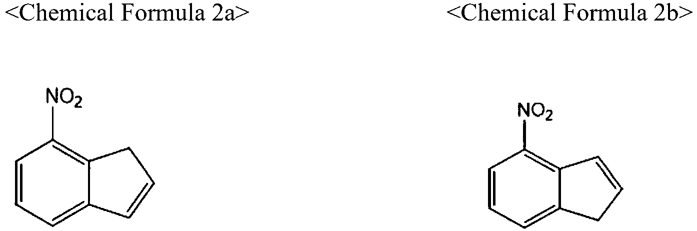

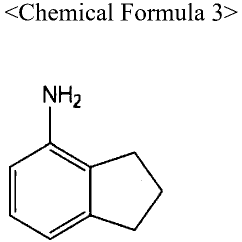

In one example, the step (Sa) may include a step of performing a decarboxylation reaction on compound 1 using palladium (II) acetate and copper (II) acetate.

In one example, the step (Sb) may include a step of performing a catalytic (catalyst) hydrogenation reaction on at least one of compounds 2a and 2b.

In one example, the step (Sb) may include a step of performing a catalytic (catalyst) hydrogenation reaction for a mixture of compounds 2a and 2b.

In one example, the catalyst may be Pd/C.

In one example, the step (Sb) may prepare 4-aminoindan from compounds 2a and 2b without a separate purification process. In the step (Sb), only 4-aminoindan is selectively prepared as a product.

In one example, the step (Sa) may include a step of reacting a compound 4 represented by chemical formula 4 to prepare the compound 1.

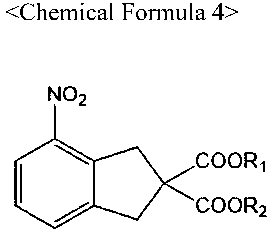

In chemical formula 4, R₁ and R₂ are each independently linear or branched C1-C6 alkyl, C3-C10 cycloalkyl, C6-C12 aryl, 4- to 7-membered heterocycloalkyl, or 5- to 7-membered heteroaryl.

In this case, the "alkyl" may be methyl, ethyl, propyl, isopropyl, etc., the "cycloalkyl" may be cyclobutyl, cyclopentyl, etc., and the "aryl" may be phenyl, biphenyl, naphthyl, etc. The "heterocycloalkyl" may be oxetanyl, piperidinyl, etc., and the "heteroaryl" may be pyridinyl, pyrimidinyl, etc.

In one example, in chemical formula 4, R₁ and R₂ may be each independently linear or branched C1-C6 alkyl. For example, in chemical formula 4, R₁ and R₂ may be each independently C1-C2 alkyl.

In one example, the compound 4 may be obtained by reacting 1,4-dioxane with a base under a first temperature condition, adding an acid, stirring the resulting solution at a second temperature higher than the first temperature, and then precipitating (crystallizing) into a solid using a filtrate with an organic phase separated therefrom. The base may be NaOH and the acid may be HCl. According to the present invention, the compound 1 may be obtained as a solid from the compound 4, and thus the compound 1 may be prepared without a separate purification process.

In one example, the step (Sa) may include a step of reacting a compound 5 represented by chemical formula 5 to prepare the compound 4.

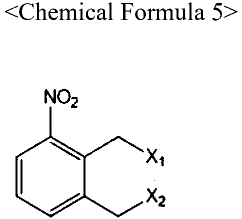

In chemical formula 5, X₁ and X₂ are each independently F, Cl, Br or I.

In one example, the step of preparing the compound 4 may include a step of reacting the compound 5 with a compound represented by chemical formula A below.

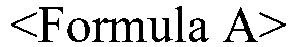

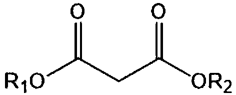

In the chemical formula A, R₁ and R₂ are each independently linear or branched C1-C6 alkyl, C3-C10 cycloalkyl, C6-C12 aryl, 4- to 7-membered heterocycloalkyl, or 5- to 7-membered heteroaryl.

In one example, the compound represented by chemical formula A may be dialkyl malonate, in which R₁ and R₂ may be each independently linear or branched C1-C5 alkyl.

In one example, the step (Sa) may include a step of reacting compound 6 represented by chemical formula 6 to prepare the compound 5.

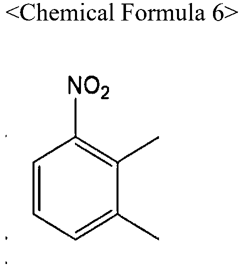

In one example, the step of preparing the compound 5 may include a step of reacting the compound 6 with a halogenated succinimide represented by chemical formula B below.

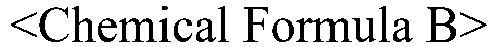

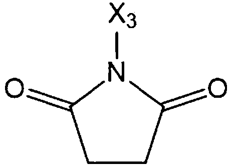

In the chemical formula B, X₃ is Cl, Br, or I.

In one example, the step (Sa) may include:
a step of preparing a compound 4 using a compound 5; and
a step of preparing compound 1 using the compound 4.

In this case, each of the steps of preparing the compound 4 and compound 1 is substantially the same as that described in the step (Sa), and a redundant detailed description will be omitted.

A method for preparing 4-aminoindan for one object of the present invention may include:
a step of preparing a compound 4 using a compound 5;
a step of preparing compound 1 using the compound 4;
a step of preparing at least one of compounds 2a and 2b using compound 1; and
a step of preparing 4-aminoindan using at least one of compounds 2a and 2b.

In this case, each step is substantially the same as those described in the steps (Sa) and (Sb), and a redundant detailed description will be omitted.

In the method for preparing a dihydroindenyl-pyrazolo[3,4-b]pyridine amine according to the present invention, the dihydroindenyl-pyrazolo[3,4-b]pyridine amine is a compound represented by chemical formula X below.

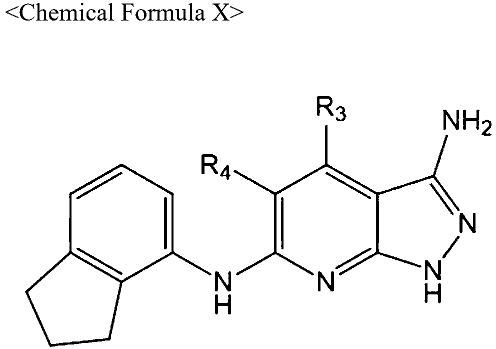

In chemical formula X, R₃ and R₄ are each independently H, OH, C1-C6 alkyl, C1-C6 haloalkyl, halogen, COOH, COO(C1-C6 alkyl), or C6-C12 aryl.

The definition of each of alkyl and aryl may be substantially the same as that described in R₁ and R₂, "haloalkyl" may be CF₃, CF₂H, CH₂CF₃, etc., and may be a functional group in which at least one of H of alkyl is substituted with halogen, and "halogen" may be F, Cl, Br, or I.

The method for preparing a dihydroindenyl-pyrazolo[3,4-b]pyridine amine according to the present invention includes:
a step of preparing at least one of compounds 2a and 2b using compound 1;
a step of preparing 4-aminoindan using at least one of compounds 2a and 2b;
a step of reacting 4-aminoindan with a compound represented by chemical formula 7 to prepare a compound represented by chemical formula 8; and
a step of performing a cyclization reaction on the compound represented by chemical formula 8 to prepare a dihydroindenyl-pyrazolo[3,4-b]pyridine amine represented by chemical formula X.

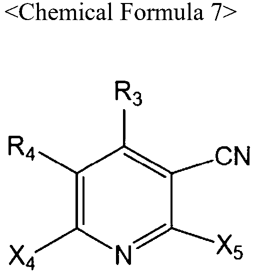

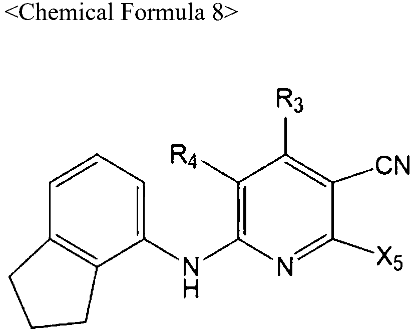

In chemical formulas 7 and 8, R₃ and R₄ is the same as defined in chemical formula X.

In chemical formulas 7 and 8, X₄ and X₅ are each independently Cl or Br.

The cyclization reaction may be performed by reacting the compound represented by chemical formula 8 with hydrazine monohydrate.

In one example, a method for preparing compound 1 may not be particularly limited, but may be prepared by using the compound 4 as a starting material as described above, and in this case, the compound 4 may be prepared by using the compound 5. In addition, the compound 5 may be prepared by using compound 6 as a starting material. A redundant detailed description will be omitted.

In one example, the present invention may provide a method for preparing N6-(2,3-dihydro-1H-inden-4-yl)-5-fluoro-1H-pyrazolo[3,4-b]pyridin-3,6-diamine.

A method for preparing N6-(2,3-dihydro-1H-inden-4-yl)-5-fluoro-1H-pyrazolo[3,4-b]pyridin-3,6-diamine according to the present invention may include:
a step of preparing at least one of compounds 2a and 2b using compound 1;
a step of preparing 4-aminoindan using at least one of compounds 2a and 2b;
a step of reacting 4-aminoindan with 2,6-dichloro-5-fluoronicotinonitrile to prepare 2-chloro-6-((2,3-dihydro-1H-inden-4-yl)amino)-5-fluoronicotinonitrile; and
a step of performing a cyclization reaction on 2-chloro-6-((2,3-dihydro-1H-inden-4-yl)amino)-5-fluoronicotinonitrile to prepare N6-(2,3-dihydro-1H-inden-4-yl)-5-fluoro-1H-pyrazolo[3,4-b]pyridine-3,6-diamine.

In one example, the step of preparing 2-chloro-6-((2,3-dihydro-1H-inden-4-yl)amino)-5-fluoronicotinonitrile may use dimethyl sulfoxide (DMSO) and N,N-diisopropylethylamine (DIPEA) when reacting 4-aminoindan with 2,6-dichloro-5-fluoronicotinonitrile. The reaction under the solvent condition may easily obtain 2-chloro-6-((2,3-dihydro-1H-inden-4-yl)amino)-5-fluoronicotinonitrile without using a harmful substance such as NMP or 2-methoxyethanol.

In this case, compound 1 in the method for preparing N6-(2,3-dihydro-1H-inden-4-yl)-5-fluoro-1H-pyrazolo[3,4-b]pyridin-3,6-diamine may be prepared through a step of preparing a compound 5 using compound 6; a step of preparing a compound 4 using the compound 5; and a step of preparing compound 1 using the compound 4. A redundant detailed description will be omitted.

According to the method for preparing the dihydroindenyl-pyrazolo[3,4-b]pyridine amine of the present invention, 4-aminoindan may be mass-produced at a high yield, and thus a preparation yield of the dihydroindenyl-pyrazolo[3,4-b]pyridine amine using 4-aminoindan as an essential reactant may also be at least 70% or higher, which may be remarkably improved compared to the related art. In addition, the dihydroindenyl-pyrazolo[3,4-b]pyridine amine may be obtained at a high yield.
1) The present invention provides a method for preparing 4-aminoindan, the method including: (Sa) a step of reacting compound 1 represented by chemical formula 1 to prepare at least one of compound 2a represented by chemical formula 2a and compound 2b represented by chemical formula 2b; and (Sb) a step of reacting at least one of compounds 2a and 2b to prepare 4-aminoindan represented by chemical formula 3: 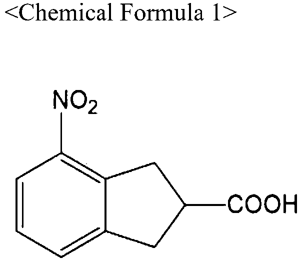 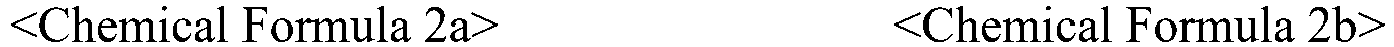 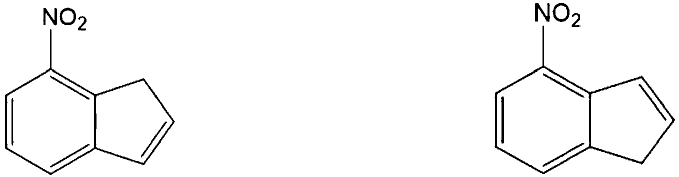 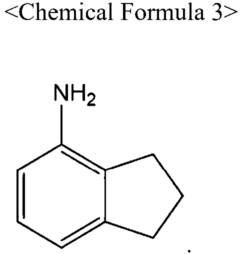
2) In 1), the step (Sb) may comprise a step of performing a catalytic (catalyst) hydrogenation reaction on at least one of compounds 2a and 2b.
3) In 2), the catalyst may be Pd/C.
4) In any one of 1) to 3), the step (Sa) may comprise a step of performing a decarboxylation reaction on compound 1 using palladium (II) acetate and copper (II) acetate.
5) In any one of 1) to 4), the step (Sa) may comprise a step of reacting a compound 4 represented by chemical formula 4 to prepare the compound 1: 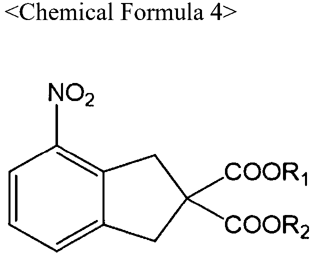 In chemical formula 4, R₁ and R₂ are each independently linear or branched C1-C6 alkyl, C3-C10 cycloalkyl, C6-C12 aryl, 4- to 7-membered heterocycloalkyl, or 5- to 7-membered heteroaryl.
6) In 5), the step (Sa) may comprise a step of preparing the compound 4 using a compound 5 represented by chemical formula 5: 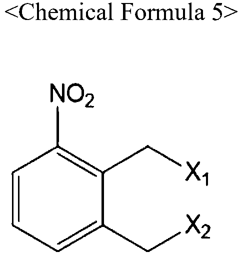 In chemical formula 5, X₁ and X₂ are each independently F, Cl, Br, or I.
7) In 6), the step of preparing the compound 4 may comprise a step of reacting the compound 5 with a compound represented by chemical formula A: 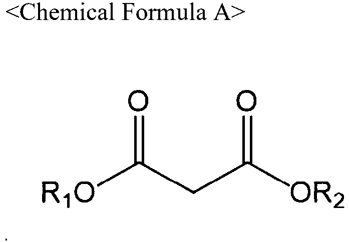 In chemical formula A, R₁ and R₂ are each independently linear or branched C1-C6 alkyl, C3-C10 cycloalkyl, C6-C12 aryl, 4- to 7-membered heterocycloalkyl, or 5- to 7-membered heteroaryl.
8) In 6) or 7), the compound 4 may be obtained as a solid crystal in the step of preparing the compound 4.
9) The present invention may provide a method for preparing 4-aminoindan, the method comprising: a step of reacting a compound 5 to prepare a compound 4 represented by chemical formula 4; a step of reacting the compound 4 to prepare compound 1 represented by chemical formula 1; a step of reacting compound 1 to prepare at least one of compound 2a represented by chemical formula 2a and compound 2b represented by chemical formula 2b; and a step of reacting at least one of compounds 2a and 2b to prepare 4-aminoindan represented by chemical formula 3: 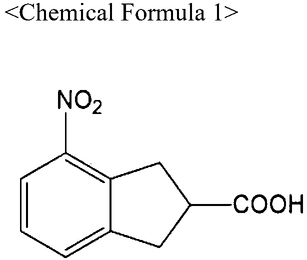 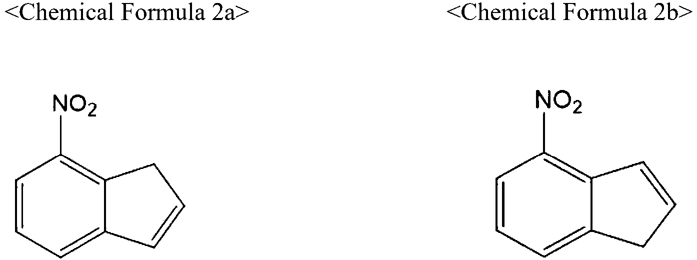 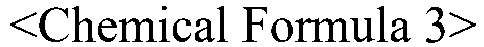 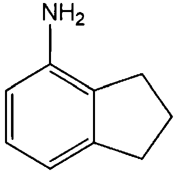 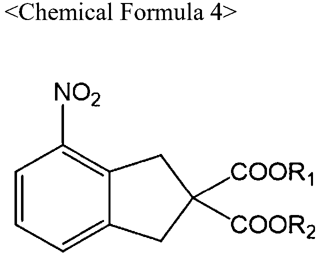 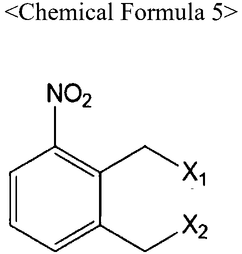 In chemical formula 4, R₁ and R₂ are each independently linear or branched C1-C6 alkyl, C3-C10 cycloalkyl, C6-C12 aryl, 4- to 7-membered heterocycloalkyl, or 5- to 7-membered heteroaryl, and in chemical formula 5, X₁ and X₂ are each independently F, Cl, Br, or I.
10) In 9), the step of preparing 4-aminoindan may comprise a step of performing a catalytic (catalyst) hydrogenation reaction on at least one of compounds 2a and 2b.
11) In 9) or 10), the step of preparing at least one of compounds 2a and 2b may comprise a step of performing a decarboxylation reaction on compound 1 using palladium (II) acetate and copper (II) acetate.
12) In any one of 9) to 11), the step of preparing the compound 4 may comprise a step of reacting the compound 5 with dialkyl malonate.
13) The present invention provides a method for preparing a dihydroindenyl-pyrazolo[3,4-b]pyridine amine, the method comprising: a step of reacting compound 1 represented by chemical formula 1 to prepare at least one of compound 2a represented by chemical formula 2a and compound 2b represented by chemical formula 2b; a step of reacting at least one of compounds 2a and 2b to prepare 4-aminoindan represented by chemical formula 3; a step of reacting 4-aminoindan with a compound represented by chemical formula 7 to prepare a compound represented by chemical formula 8; and a step of performing a cyclization reaction on the compound represented by chemical formula 8 to prepare a dihydroindenyl-pyrazolo[3,4-b]pyridine amine represented by chemical formula X: 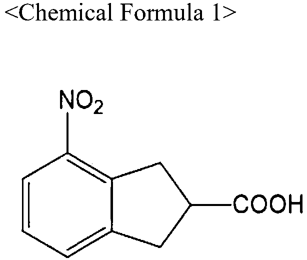 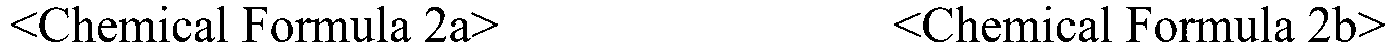 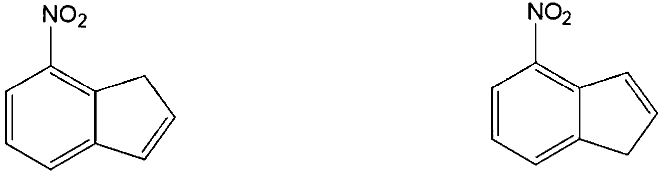 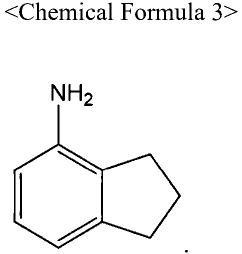 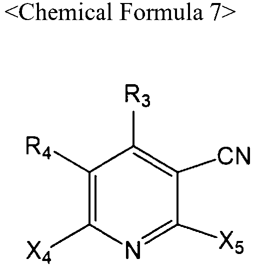 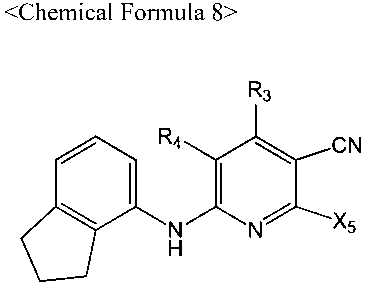 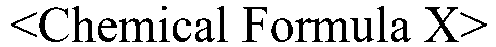 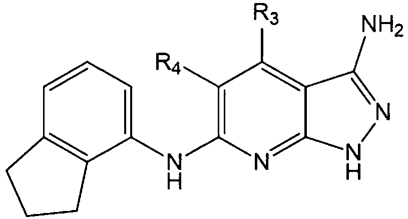 In chemical formulas 7, 8, and X, R₃ and R₄ are each independently H, OH, C1-C6 alkyl, C1-C6 haloalkyl, halogen, COOH, COO(C1-C6 alkyl), or C6-C12 aryl, and in chemical formulas 7 and 8, X₄ and X₅ are each independently Cl or Br.
14) In 13), the dihydroindenyl-pyrazolo[3,4-b]pyridine amine may be N6-(2,3-dihydro-1H-inden-4-yl)-5-fluoro-1H-pyrazolo[3,4-b]pyridin-3,6-diamine.

### ADVANTAGEOUS EFFECTS

According to the method for preparing 4-aminoindan and the method for preparing the dihydroindenyl-pyrazolo[3,4-b]pyridine amine of the present invention described above, 5-aminoindan cannot be produced at all during the preparation process, and thus the process of separately isolating 4-aminoindan can be fundamentally omitted. In addition, each step can exhibit a preparation yield of at least 70% or more, and thus an overall preparation yield of 4-aminoindan can be remarkably improved. Accordingly, 4-aminoindan can be mass-produced at a high yield, and the dihydroindenyl-pyrazolo[3,4-b]pyridine amine can also be prepared at a high yield and with a high purity.

### DESCRIPTION OF DRAWINGS

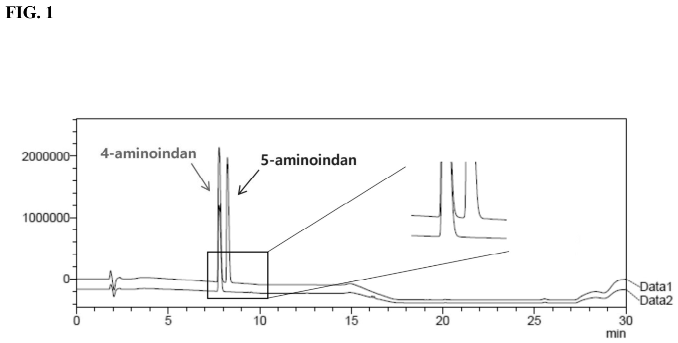

FIG. 1 is a view showing an HPLC analysis graph of a product obtained in step 4 of Example 1 of the present invention.

### DETAILED DESCRIPTION OF THE EMBODIMENTS

Terms used in the present application are used only to describe a certain example and are not intended to limit the present invention. In the present application, terms such as "include," "have" or the like shall be intended to designate a presence of features, steps, operations, components, or combinations thereof described herein, and shall not be construed to exclude a possible presence or addition of one or more other features, steps, operations, components, or combinations thereof in advance. All the terms used herein including technical or scientific terms have the same meaning as commonly understood by those ordinary skilled in the art, to which the present invention pertains, unless defined otherwise.

### Example 1: Method for preparing 4-aminoindan

### (Step 1) Preparation of dimethyl 4-nitro-1H-inden-2,2(3H)-dicarboxylate (compound 4)

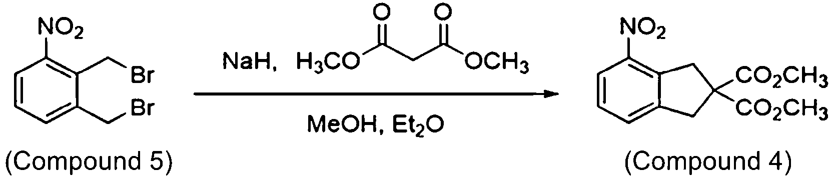

A weight (unit: g) of compound 5 to be used as a starting material was determined to be 1 eq., and then methanol (volume (mL) = 1.6 times the weight (g) corresponding to 1 eq. of compound 5) and diethyl ether (volume (mL) = 4.8 times the weight (g) corresponding to 1 eq. of compound 5) were added into a reactor filled with nitrogen at room temperature (20 to 30°C), and then sodium hydride (60%) (2.4 eq.) was added into the resulting mixture and stirred for five minutes to obtain a suspension. Dimethyl malonate (1.2 eq.) and compound 5 (1 eq.) obtained according to the process of the step 1 were sequentially and slowly added into the obtained suspension. The resulting mixture was stirred at 20 to 30°C for two hours. The reaction solution was filtered and the filtrate was concentrated. After adding dichloromethane (DCM) to the concentrated residue, the precipitated solid was filtered. Water was added into the filtrate and then stirred, and an aqueous phase was discarded and an organic phase was collected and concentrated. The concentrated residue was purified by column chromatography to obtain the title compound 4 (gray solid, yield 81.35%).

¹H NMR : 8.034-8.018 (1H, m), 7.697-7.687 (1H, m), 7.505-7.482 (1H, m), 3.957 (2H, s), 3.715 (6H, s), 3.632 (2H, s).

¹³C NMR : 170.95, 144.71, 143.93, 136.03, 130.73, 128.76, 122.51, 58.91, 53.18, 40.80, 39.48.

### (Step 2) Preparation of 4-nitro-2,3-dihydro-1H-inden-2-carboxylic acid (compound 1)

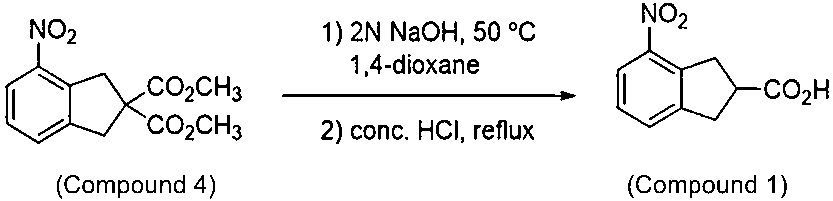

Compound 4 (1 eq.) obtained according to the process of the step 1 and 1,4-dioxane (volume (mL) = eight times the weight (g) corresponding to 1 eq. of compound 4) were added into a reactor filled with nitrogen at room temperature (20 to 30°C), and then 2 N NaOH (volume (mL) = eight times the weight (g) corresponding to 1 eq. of compound 4) was added into the resulting mixture and stirred for five minutes. A temperature was raised to 50°C and then stirred for two hours. Subsequently, after cooling to 20 to 30°C, the pH of the mixed solution was adjusted to 1 with conc. HCl. The mixed solution of which pH was adjusted to 1 was heated to 100°C and then stirred for six hours. After cooling to 20 to 30°C, water (volume (mL) = 18 times the weight (g) corresponding to 1 eq. of an initial input in step 2 of compound 4) and ethyl acetate (volume (mL) = 18 times the weight (g) corresponding to 1 eq. of an initial input in step 2 of compound 4) were added thereto and stirred for ten minutes. After an organic phase was separated, MgSO₄ was added thereto to carry out dehydration and filtration, and then the filtered filtrate was concentrated. After the concentrated residue was added into the reactor, ethyl acetate (volume (mL) = five times the weight (g) corresponding to 1 eq. of an initial input in step 2 of compound 4) was added, and a temperature was raised to 50°C to be dissolved. When solid precipitation was confirmed after slowly cooling the mixed solution, heptane (volume (mL) = 20 times the weight (g) corresponding to 1 eq. of an initial input of compound 4) was added, and the suspension was stirred at 20 to 30°C for one hour. The product was filtered and washed with heptane (volume (mL) = ten times the weight (g) corresponding to 1 eq. of an initial input in step 2 of compound 4), and then vacuum-dried for 12 hours to obtain the title compound 1 (brown powder, yield 87.90%).

¹H NMR : 12.697 (1H, m), 7.986 (1H, d, J=8.3 Hz), 7.663 (1H, d, J=4.89 Hz), 7.453-7.439 (1H, m), 3.633-3.500 (2H, m), 3.386-3.210 (3H, m)

¹³C NMR : 175.699, 172.365, 146.004, 138.039, 130.592, 128.181, 122.009, 42.119, 36.649, 35.482.

### (Step 3) Preparation of nitro-1H-indene (compounds Sa, Sb)

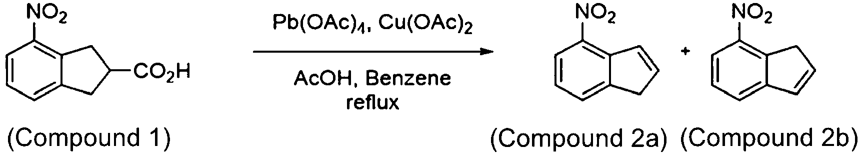

Compound 1 (1 eq.) obtained according to the process of the step 2, Pb(OAc)₄ (0.806 eq.), Cu(OAc)₂·H₂O (0.177 eq.), benzene, and acetic acid (benzene : AcOH = 20 : 1, volume (mL) = 12.5 times the weight (g) corresponding to 1 eq. of compound 1) were added into a reactor filled with nitrogen at room temperature (20 to 30°C). A temperature was raised to 100 to 110°C and stirred at the same temperature for three hours. The reaction solution was concentrated and purified via column chromatography (hexane : ethyl acetate = 5 : 1) to prepare a mixture of the title compounds 2a and 2b (yellow powder, yield 70.51%).

¹H NMR (500 MHz, DMSO-d6) δ ppm 2.500 (dt, *J*=3.417, 1.709 Hz, 1 H) 3.595 - 3.656 (m, 1 H) 3.862 - 3.920 (m, 2 H) 6.772 - 6.883 (m, 1 H) 6.999 - 7.102 (m, 1 H) 7.336 - 7.381 (m, 1 H) 7.407 - 7.473 (m, 1 H) 7.496 - 7.538 (m, 1 H) 7.542 - 7.549 (m, 1 H) 7.548 - 7.622 (m, 1 H) 7.856 (d, J=7.5 Hz, 1 H) 7.890 (d, *J*=7.323 Hz, 1 H) 8.005 (d, J=8.055 Hz, 1 H) 8.087 (d, *J*=8.299 Hz, 1 H)

¹³C NMR : 147.86, 146.83, 144.30, 141.88, 139.40, 138.71, 137.25, 130.50, 129.85, 129.60, 128.23, 128.00, 127.21, 125.42, 121.89, 119.74, 40.63, 39.25.

### (Step 4) Preparation of 4-aminoindan (compound 3)

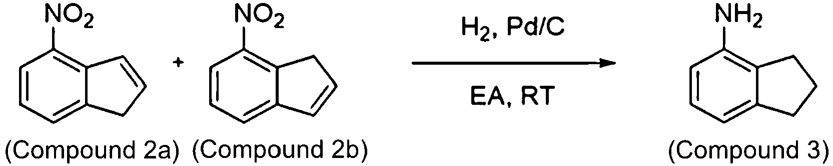

A mixture (1 eq.) of compounds 2a and 2b obtained according to the process of the step 3 and a sufficient amount of ethyl acetate were added into a reactor filled with nitrogen at room temperature. Subsequently, 10% Pd/C (20 wt%) was added into the mixed solution, and a balloon containing H₂ gas was added into the reaction solution to inject H₂ gas. The resulting mixture was stirred at 24 to 30°C for two hours and filtered to remove impurities, and then the filtrate was concentrated to obtain the title compound 3 (brown oil, yield 86.72%).

¹H NMR : 7.026-6.996 (1H, m), 6.727-6.713 (1H, m), 6.527-6.511 (1H, m), 2.935 (2H, t, *J*=7.33 Hz), 2.750 (2H, t, *J*=7.33 Hz), 2.152-2.092 (2H, m)

¹³C NMR : 144.29, 144.02, 127.09, 126.83, 112.10, 111.37, 32.80, 29.62, 24.23.

### Comparative Example 1

Step A and step B of "Intermediate R4" of international publication WO2019/211463 were sequentially performed to prepare 4-aminoindan.

A yield of the product (nitroindan) obtained according to step A of "Intermediate R4" was 53.51%, and a yield of the product (aminoindan) obtained according to step B of "Intermediate R4" was 42.55%.

As a result of analyzing the product (nitroindan) obtained according to step A of "Intermediate R4" via HPLC, it was confirmed that 4-nitroindan and 5-nitroindan are mixed at a ratio of 35 : 75 to 40 : 60. When 4-aminoindan and 5-aminoindan were mixed at the ratio range even in the product (aminoindan) obtained by directly applying the product (nitroindan) obtained according to step A of "Intermediate R4" to step B, and 4-aminoindan was obtained from the product obtained according to step B, that is, in the product obtained through steps A and B using indan as a starting material, a preparation yield of only 4-aminoindan excluding 5-aminoindan was 7.9 to 9.1%.

### Evaluation 1 - Confirmation of inclusion of 5-aminoindan

An HPLC analysis was performed on the product obtained in step 5 of Example 1 of the present invention. As a control sample, a mixture of 4-aminoindan and 5-aminoindan was used. The results of the HPLC analysis are shown in FIG. 1.

Referring to FIG. 1, it can be confirmed that each peak of 4-aminoindan and 5-aminoindan appears in the mixture of 4-aminoindan and 5-aminoindan (see a graph of data 1). On the contrary, in the product obtained in step 5 of Example 1 of the present invention, it can be confirmed that only one peak appears (see a graph of data 2), and as a result of comparing the same with the peaks of the control sample, a corresponding peak may be a peak for 4-aminoindan.

Accordingly, with regard to the product obtained in step 5 of Example 1 of the present invention, it can be confirmed that only 4-aminoindan is selectively obtained at 100% even without a separate purification process in step 5.

### Example 2: Preparation of N6-(2,3-dihydro-1H-inden-4-yl)-5-fluoro-1H-pyrazolo[3,4-b]pyridin-3,6-diamine

### Steps 1 to 4: Preparation of 4-aminoindan

4-aminoindan was prepared according to substantially the same process as described in steps 1 to 4 of Example 1.

### Step 5: Preparation of 2-chloro-6-((2,3-dihydro-1H-inden-4-yl)amino)-5-fluoronicotinonitrile

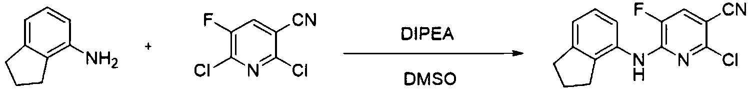

4-aminoindan obtained in the step 4, 2,6-dichloro-5-fluoronicotinonitrile, and dimethyl sulfoxide (DMSO) were added into a reactor dried and filled with nitrogen at room temperature (20 to 30°C), and then N,N-diisopropylethylamine (DIPEA) was added thereto. In this case, an amount of 2,6-dichloro-5-fluoronicotinonitrile added was 1.58 times the weight (kg), an amount of DMSO added was 11 times the weight (kg), and an amount of DIPEA added was 1.18 times the weight (kg) based on the weight (kg) of 4-aminoindan.

A temperature inside the reactor was raised to 100±5°C and stirred for three hours, and then a temperature inside the reactor was cooled to 25±5°C. When a temperature inside the reactor reached 25±5°C, distilled water was directly added without temperature control. In this case, an amount of the distilled water used was ten times the weight (kg) based on the weight (kg) of initially added 4-aminoindan. After the distilled water was completely added, a temperature inside the reactor was cooled to 25±5°C and stirred for 15 minutes. An obtained crystallized liquid was filtered and washed with distilled water (20 times the weight (kg) of an initial input weight (kg) of 4-aminoindan). An obtained filtered cake was dried under reduced pressure while an external temperature was set to 40°C or less. Accordingly, 2-chloro-6-((2,3-dihydro-1H-inden-4-yl)amino)-5-fluoronicotinonitrile (hereinafter, referred to as crude-WA) was obtained as crude.

Subsequently, dichloromethane (DCM) and the crude-WA obtained in the above step were added into a reactor dried and filled with nitrogen at room temperature (20 to 30°C). In this case, an amount of DMC added was 13.3 times the weight (kg) based on the weight (kg) of the crude-WA.

A temperature inside the reactor was raised to 40±5°C, and it was confirmed whether or not all of the crude-WA in the reactor was dissolved. The resulting mixture was further stirred at the same temperature for 15 minutes. After that, the temperature inside the reactor was cooled to 25±5°C, and heptane was added when crystals were precipitated. An amount of heptane added was 20.52 times the weight (kg) based on the weight (kg) of the crude-WA added into the reactor. After the heptane was completely added, a temperature inside the reactor was cooled to 0±5°C. After the cooling was completed, the resulting product was cooled and aged at the same temperature for one hour, and the obtained crystallized liquid was filtered and washed with heptane. In this case, an amount of heptane used was 10.26 times the weight (kg) based on the weight (kg) of the crude-WA initially added into the reactor. An obtained filtered cake was dried under reduced pressure while an external temperature was set to 40°C or less. Accordingly, 2-chloro-6-((2,3-dihydro-1H-inden-4-yl)amino)-5-fluoronicotinonitrile was obtained (100 g of 4-aminoindan used in step 5, 181.73 g of 2-chloro-6-((2,3-dihydro-1H-inden-4-yl)amino)-5-fluoronicotinonitrile obtained, yield 88.25%, purity 99.6%).

### Step 6: Preparation of N6-(2,3-dihydro-1H-inden-4-yl)-5-fluoro-1H-pyrazolo[3,4-b]pyridin-3,6-diamine

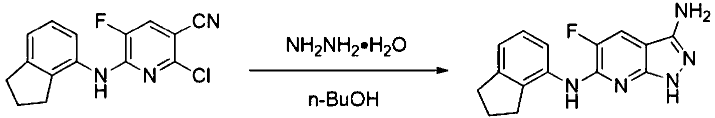

2-chloro-6-((2,3-dihydro-1H-inden-4-yl)amino)-5-fluoronicotinonitrile obtained in the step 5 and n-BuOH were added into a reactor dried and filled with nitrogen, and then hydrazine monohydrate was added thereto. In this case, an amount of n-BuOH added was 4.05 times the weight (kg) and an amount of hydrazine monohydrate added was 1.16 times the weight (kg) based on the weight (kg) of 2-chloro-6-((2,3-dihydro-1H-inden-4-yl)amino)-5-fluoronicotinonitrile.

After raising a temperature inside the reactor to 105±5°C, the resulting mixture was stirred for seven hours while maintaining the internal temperature. Subsequently, the inside of the reactor was cooled to 0±5°C and then further stirred for one hour. An obtained crystallized liquid was filtered and washed with distilled water (ten times the weight (kg) based on the weight (kg) of 2-chloro-6-((2,3-dihydro-1H-inden-4-yl)amino)-5-fluoronicotinonitrile added into the reactor). A filtered cake was dried under reduced pressure while an external temperature was set to 40°C or less. Accordingly, N6-(2,3-dihydro-1H-inden-4-yl)-5-fluoro-1H-pyrazolo[3,4-b]pyridine-3,6-diamine (hereinafter referred to as crude-product) was obtained as crude.

Subsequently, the crude-product obtained in the above step and the DMSO were added into a reactor dried and filled with nitrogen at room temperature (20 to 30°C). In this case, an amount of the DMSO added was 5.5 times the weight (kg) based on the weight (kg) of the crude-product. When it was confirmed that all crude-products inside the reactor were dissolved, distilled water was added dropwise while maintaining the internal temperature at 25±5°C. In this case, an amount of distilled water added was three times the weight (kg) based on the weight (kg) of the crude-product added into the reactor. After the distilled water was completely added, the resulting mixture was aged and stirred for one hour while maintaining a temperature inside the reactor to 25±5°C. An obtained crystallized liquid was filtered and washed with distilled water (10 times the weight (kg) based on the weight (kg) of the crude-product added into the reactor). An obtained filtered cake was dried under reduced pressure while an external temperature was set to 40°C or less. Accordingly, N6-(2,3-dihydro-1H-inden-4-yl)-5-fluoro-1H-pyrazolo[3,4-b]pyridine-3,6-diamine was prepared (139.64 g of N6-(2,3-dihydro-1H-inden-4-yl)-5-fluoro-1H-pyrazolo[3,4-b]pyridin-3,6-diamine was obtained by directly using the final product obtained in step 6, yield 82.73% and purity 99.7%).

¹H NMR : 11.426 (s, 1 H), 11.392-11.468 (m, 1 H), 8.315 (s, 1 H), 7.731 (s, 1 H), 7.678-7.761 (m, 1 H), 7.359 (d, *J*=7.816 Hz, 1 H), 7.112 (t, *J*=7.572 Hz, 1 H), 7.011 (d, *J*=7.328 Hz, 1 H), 6.978-7.046 (m, 1 H), 5.213 (s, 2 H), 2.891 (t, *J*=7.328 Hz, 2 H), 2.754 (t, *J*=7.328 Hz, 2 H), 1.967 (quin, *J*=7.328 Hz, 2 H).

### Evaluation 2 - Quality and yield

It was confirmed that the purity of N6-(2,3-dihydro-1H-inden-4-yl)-5-fluoro-1H-pyrazolo[3,4-b]pyridine-3,6-diamine, which is the final product obtained in Example 2, is 99.7%, which is very excellent. In particular, it was also confirmed that the purity obtained by the method for preparing N6-(2,3-dihydro-1H-inden-4-yl)-5-fluoro-1H-pyrazolo[3,4-b]pyridine-3,6-diamine according to the present invention is improved compared to the purity of N6-(2,3-dihydro-1H-inden-4-yl)-5-fluoro-1H-pyrazolo[3,4-b]pyridine-3,6-diamine prepared by preparing 4-aminoindan according to the conventional method and using the same.

In addition, since an overall preparation yield of the method for preparing N6-(2,3-dihydro-1H-inden-4-yl)-5-fluoro-1H-pyrazolo[3,4-b]pyridine-3,6-diamine according to the present invention uses the method for preparing the 4-aminoindan of the present invention with remarkably improved productivity compared to the conventional method, it can be confirmed that the overall preparation yield of the method according to the present invention is significantly improved at least four times or more compared to the preparation yield of the conventional method for preparing 4-aminoindan and using the same to prepare N6-(2,3-dihydro-1H-inden-4-yl)-5-fluoro-1H-pyrazolo[3,4-b]pyridine-3,6-diamine.

Even in a method for synthesizing N6-(2,3-dihydro-1H-inden-4-yl)-5-fluoro-1H-pyrazolo[3,4-b]pyridine-3,6-diamine using 4-aminoindan as a starting material in the same way, in the case of obtaining 4-aminoindan according to the present invention and preparing N6-(2,3-dihydro-1H-inden-4-yl)-5-fluoro-1H-pyrazolo[3,4-b]pyridine-3,6-diamine, it was confirmed that the preparation yield is excellent compared to the case of obtaining 4-aminoindan by the conventional method and preparing N6-(2,3-dihydro-1H-inden-4-yl)-5-fluoro-1H-pyrazolo[3,4-b]pyridine-3,6-diamine.

The present invention has been described with reference to preferred exemplary embodiments herein, but it will be understood by those skilled in the art that the present invention may be variously changed and modified without departing from the spirit and field of the present invention, as described in the following scope of patent claims.

## Claims

1. A method for preparing 4-aminoindan, the method comprising:
(Sa) a step of reacting compound 1 represented by chemical formula 1 to prepare at least one of compound 2a represented by chemical formula 2a and compound 2b represented by chemical formula 2b; and
(Sb) a step of reacting at least one of compounds 2a and 2b to prepare 4-aminoindan represented by chemical formula 3:

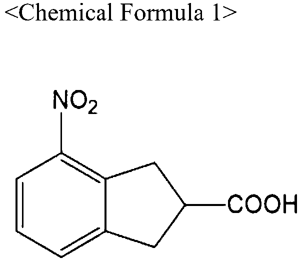

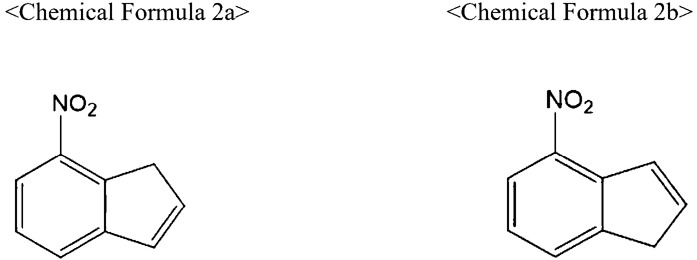

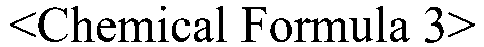

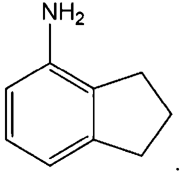

2. The method of claim 1, wherein the step (Sb) comprises a step of performing a catalytic (catalyst) hydrogenation reaction on at least one of compounds 2a and 2b.

3. The method of claim 2, wherein the catalyst is Pd/C.

4. The method of claim 1, wherein the step (Sa) comprises a step of performing a decarboxylation reaction on compound 1 using palladium (II) acetate and copper (II) acetate.

5. The method of claim 1, wherein the step (Sa) comprises a step of reacting a compound 4 represented by chemical formula 4 to prepare the compound 1: 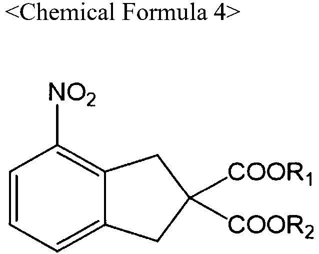
wherein in chemical formula 4,
R₁ and R₂ are each independently linear or branched C1-C6 alkyl, C3-C10 cycloalkyl, C6-C12 aryl, 4- to 7-membered heterocycloalkyl, or 5- to 7-membered heteroaryl.

6. The method of claim 5, wherein the step (Sa) comprises a step of preparing the compound 4 using a compound 5 represented by chemical formula 5: 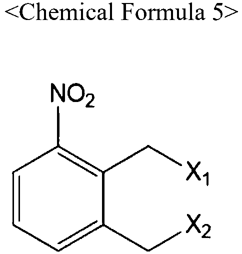 wherein in chemical formula 5, X₁ and X₂ are each independently F, Cl, Br or I.

7. The method of claim 6, wherein the step of preparing the compound 4 comprises a step of reacting the compound 5 with a compound represented by chemical formula A: 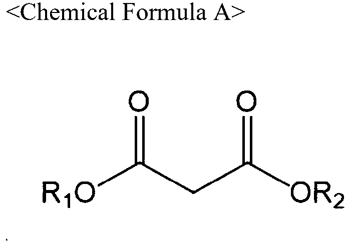
wherein in chemical formula A,
R₁ and R₂ are each independently linear or branched C1-C6 alkyl, C3-C10 cycloalkyl, C6-C12 aryl, 4- to 7-membered heterocycloalkyl, or 5- to 7-membered heteroaryl.

8. The method of claim 6, wherein the compound 4 is obtained as a solid crystal in the step of preparing the compound 4.

9. A method for preparing 4-aminoindan, the method comprising:
a step of reacting a compound 5 to prepare a compound 4 represented by chemical formula 4;
a step of reacting the compound 4 to prepare compound 1 represented by chemical formula 1;
a step of reacting compound 1 to prepare at least one of compound 2a represented by chemical formula 2a and compound 2b represented by chemical formula 2b; and
a step of reacting at least one of compounds 2a and 2b to prepare 4-aminoindan represented by chemical formula 3: 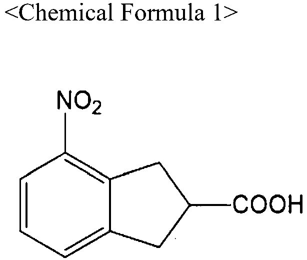 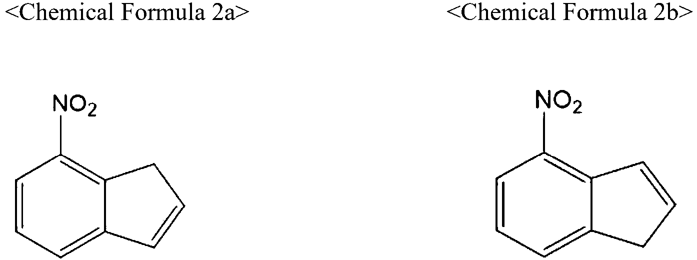 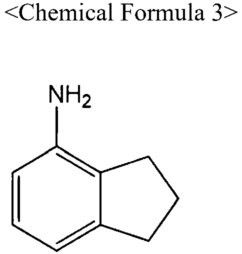 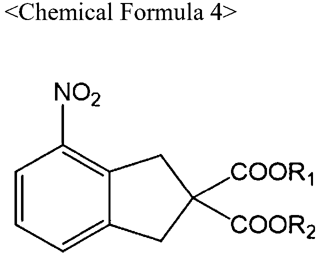 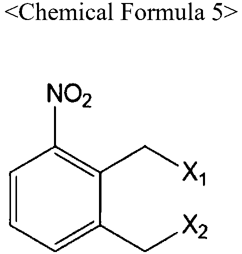
wherein in chemical formula 4, R₁ and R₂ are each independently linear or branched C1-C6 alkyl, C3-C10 cycloalkyl, C6-C12 aryl, 4- to 7-membered heterocycloalkyl, or 5- to 7-membered heteroaryl, and
in chemical formula 5, X₁ and X₂ are each independently F, Cl, Br or I.

10. The method of claim 9, wherein the step of preparing 4-aminoindan comprises a step of performing a catalytic (catalyst) hydrogenation reaction on at least one of compounds 2a and 2b.

11. The method of claim 9, wherein the step of preparing at least one of compounds 2a and 2b comprises a step of performing a decarboxylation reaction on compound 1 using palladium (II) acetate and copper (II) acetate.

12. The method of claim 9, wherein the step of preparing the compound 4 comprises a step of reacting the compound 5 with dialkyl malonate.

13. A method for preparing a dihydroindenyl-pyrazolo[3,4-b]pyridine amine, the method comprising:
a step of reacting compound 1 represented by chemical formula 1 to prepare at least one of compound 2a represented by chemical formula 2a and compound 2b represented by chemical formula 2b;
a step of reacting at least one of compounds 2a and 2b to prepare 4-aminoindan represented by chemical formula 3;
a step of reacting 4-aminoindan with a compound represented by chemical formula 7 to prepare a compound represented by chemical formula 8; and
a step of performing a cyclization reaction on the compound represented by chemical formula 8 to prepare a dihydroindenyl-pyrazolo[3,4-b]pyridine amine represented by chemical formula X: 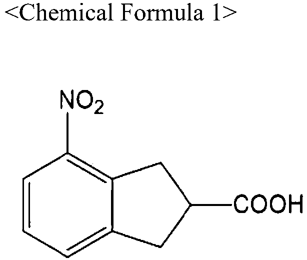 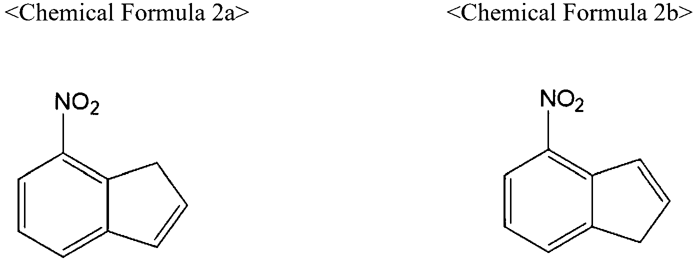 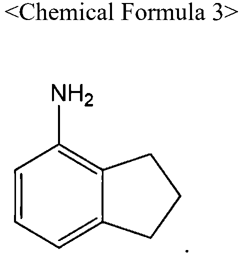 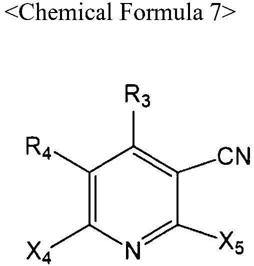 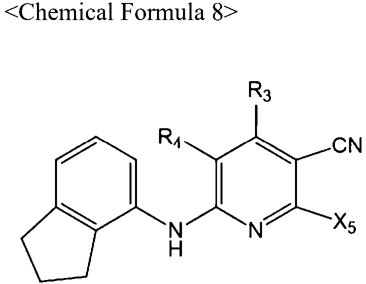 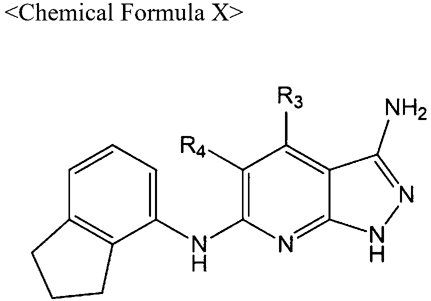
wherein in chemical formulas 7, 8 and X, R₃ and R₄ are each independently H, OH, C1-C6 alkyl, C1-C6 haloalkyl, halogen, COOH, COO(C1-C6 alkyl), or C6-C12 aryl, and
in chemical formulas 7 and 8, X₄ and X₅ are each independently Cl or Br.

14. The method of claim 13, wherein the dihydroindenyl-pyrazolo[3,4-b]pyridine amine is N6-(2,3-dihydro-1H-inden-4-yl)-5-fluoro-1H-pyrazolo[3,4-b]pyridin-3,6-diamine.
